# EUROPEAN PATENT APPLICATION

(11) **EP 3 124 024 A1**
(43) Date of publication of application: **01.02.2017**
(21) Application number: 15769927.3
(22) Date of filing: 26.03.2015
(51) Int. Cl.: A61K 31/4152, A61K 31/519, A61K 45/00, A61P 27/02, A61P 27/06, A61P 35/00, A61P 43/00

(54) **MEDICINAL COMPOSITION INHIBITING NEOVASCULARIZATION PROLIFERATION FACTOR**

(30) Priority: 27.03.2014 JP 2014066716
(71) Applicant: Kyoto University, Kyoto-shi, Kyoto 606-8501 (JP); National University Corporation Tokyo Medical and Dental University, Bunkyo-ku Tokyo 113-8510 (JP)
(72) Inventor: HAGIWARA, Masatoshi, Kyoto 606-8501 (JP); MOROOKA, Satoshi, Kyoto 606-8501 (JP); HOSOYA, Takamitsu, Tokyo 113-8510 (JP); YOSHIDA, Suguru, Tokyo 113-8510 (JP)
(74) Representative: Jones, Elizabeth Louise
(86) International application number: PCT/JP2015/059461
(87) International publication number: WO 2015/147204

(57) **Abstract**

A pharmaceutical composition for neovascular diseases, a pharmaceutical composition for inhibiting an angiogenic growth factor, and use of these pharmaceutical compositions are provided.

In one or more embodiments, a pharmaceutical composition contains as an active ingredient a low molecular weight compound that is able to suppress the expression of the VEGF gene in cells or to reduce the production of VEGF protein from cells. In one or more embodiments, a pharmaceutical composition contains as an active ingredient a compound expressed by the following formula (I) or a prodrug thereof or pharmaceutically acceptable salts thereof

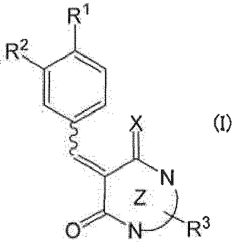

## Description

### Technical Field

The present disclosure relates to a pharmaceutical composition for neovascular diseases, a pharmaceutical composition for inhibiting an angiogenic growth factor (including VEGF), and use of these pharmaceutical compositions.

### Background Art

Diseases involving neovascularization are known as, e.g., retinal and choroidal neovascular diseases and cancer. One of the typical symptoms of the retinal and choroidal neovascular diseases is age-related macular degeneration. Age-related macular degeneration is deterioration of the macula, which is the central area of the retina, with aging. In Japan, age-related macular degeneration is the third most common cause of blindness, and affects approximately 690,000 patients. The number of patients is growing with increasing age. Pathologic choroidal neovascularization (CNV) is considered to cause wet age-related macular degeneration. On the other hand, cancer makes up about 30% of deaths in Japan, and the incidence rate of cancer increases with age. Most cancers are solid, and solid cancer induces the formation of new blood vessels in tumors, which supply oxygen and nutrients to the tumor cells, leading to tumor expansion. Moreover, the tumor cells enter the newly formed blood vessels and are carried to a distant organ through large vessels. This is so-called metastasis. Thus, tumor angiogenesis may contribute to a worsening of cancer.

Patent Document 1 discloses a compound that inhibits SRPK (kinase) and has antiviral activity. Non-Patent Document 1 discloses that the compound taught by Patent Document 1 can suppress retinal neovascularization.

### Prior Art Documents

### Patent Documents

Patent Document 1: WO 2005/063293 A1

### Non-Patent Documents

Non-Patent Document 1: Nowak DG, Bates DO et al., J. Bio. Chem. 2010

### Disclosure of Invention

### Problem to be Solved by the Invention

In one aspect, the present disclosure provides a pharmaceutical composition for neovascular diseases, a pharmaceutical composition for inhibiting an angiogenic growth factor, and use of these pharmaceutical compositions.

### Means for Solving Problem

In one or more embodiments, the present disclosure relates to a pharmaceutical composition for neovascular diseases. The pharmaceutical composition contains as an active ingredient a low molecular weight compound that is able to suppress the expression of the VEGF gene in cells or to reduce the production of VEGF protein from cells. In one or more embodiments, the present disclosure relates to a pharmaceutical composition for inhibiting an angiogenic growth factor. The pharmaceutical composition contains as an active ingredient a low molecular weight compound that is able to suppress the expression of the VEGF gene in cells or to reduce the production of VEGF protein from cells.

In one or more embodiments, the present disclosure relates to a pharmaceutical composition for neovascular diseases. The pharmaceutical composition contains as an active ingredient a compound expressed by the following formula (I) or a prodrug thereof or pharmaceutically acceptable salts thereof 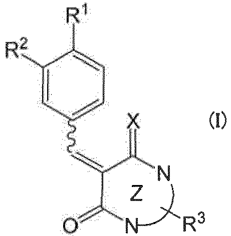 where, in the formula (I), R¹ represents a hydrogen atom, a halogen atom, a hydroxyl group, or a substituted or unsubstituted C₁₋₆ alkyl group, R² represents a hydrogen atom, a halogen atom, a hydroxyl group, a nitro group (-NO₂), a substituted or unsubstituted C₁₋₆alkyloxy group, or a substituted or unsubstituted C₁₋₆alkyl group, X represents NH or O, Z represents a 6- or 5-membered monocyclic nitrogen-containing heterocyclic ring, or a bicyclic nitrogen-containing heterocyclic ring formed by the condensation of a 6- or 5-membered nitrogen-containing heterocyclic ring and a 6- or 5-membered aliphatic ring, aromatic ring, or heterocyclic ring, R³ represents a hydrogen atom, a halogen atom, a hydroxyl group, a substituted or unsubstituted C₁₋₄alkyl group, a substituted or unsubstituted benzyl or heteroarylmethyl group, a substituted or unsubstituted aryl group, or a substituted or unsubstituted heteroaryl group, and a bond represented by a wavy line indicates a cis form, a trans form, or a mixture of a cis form and a trans form.

In one or more embodiments, the present disclosure relates to the use of a low molecular weight compound in treatment of neovascular diseases. The low molecular weight compound is able to suppress the expression of the VEGF gene in cells or to reduce the production of VEGF protein from cells.

In one or more embodiments, the present disclosure relates to the use of a low molecular weight compound in the manufacture of a pharmaceutical composition for neovascular diseases. The low molecular weight compound is able to suppress the expression of the VEGF gene in cells or to reduce the production of VEGF protein from cells.

In one or more embodiments, the present disclosure relates to a method for improving, inhibiting the development of, and/or treating neovascular diseases. The method includes administering a pharmaceutical composition containing a low molecular weight compound as an active ingredient to a subject. The low molecular weight compound is able to suppress the expression of the VEGF gene in cells or to reduce the production of VEGF protein from cells.

### Brief Description of Drawings

FIG. 1 is a graph showing an example of the results of evaluating the expression level of a vascular endothelial growth factor (VEGF) by ELISA in the culture medium of human retinal pigment epithelial cells (ARPE-19 cell line) after the administration of compounds 1 to 3.
FIG. 2 is a graph showing an example of the results of evaluating the area of choroidal neovascularization (CNV) formed in model mice with CNV after the administration of compounds 1 to 3.
FIG. 3 is a graph showing an example of the concentration dependence of a reduction in the area of CNV formed by compound 2.
FIG. 4 shows an example of the results of evaluating the area of CNV formed in CNV model mice after the application of an ophthalmic ointment containing compound 2. A of FIG. 4 is a graph that indicates an example of the area of CNV. B of FIG. 4 shows an example of fluorescence microscopy images of the choroid.

### Description of the Invention

In one aspect, the present disclosure is based on the findings that the "low molecular weight compound that is able to suppress the expression of the VEGF gene in cells or to reduce the production of VEGF protein from cells" can inhibit neovascularization, in particular, choroidal neovascularization (CNV). Thus, in one aspect, the present disclosure relates to a pharmaceutical composition for neovascular diseases, containing as an active ingredient a low molecular weight compound that is able to suppress the expression of the VEGF gene in cells or to reduce the production of VEGF protein from cells (also referred to as a "pharmaceutical composition of the present disclosure" in the following). In one or more embodiments, the pharmaceutical composition of the present disclosure inhibits an angiogenic growth factor.

### [Pharmaceutical composition for neovascular diseases]

The "neovascular diseases" in the present disclosure mean diseases involving neovascularization. In one or more embodiments, the neovascular diseases may be retinal and choroidal neovascular diseases or cancer.

In one or more embodiments, the "retinal and choroidal neovascular diseases" in the present disclosure may be age-related macular degeneration, glaucoma, occlusion of the retinal vein, or diabetic retinopathy.

In one or more embodiments, the pharmaceutical composition of this aspect is effective in prevention, improvement, inhibition of the development, and/or treatment of neovascular diseases when it is administered to a subject. In one or more embodiments, examples of the subject include mammals, humans, and mammals other than humans. Therefore, in one or more embodiment, the pharmaceutical composition of the present disclosure is a pharmaceutical composition for preventing, improving, inhibiting the development of, and/or treating neovascular diseases.

### [Active ingredient of pharmaceutical composition]

In one or more embodiments, the active ingredient of the pharmaceutical composition of the present disclosure is a low molecular weight compound that is able to suppress the expression of the vascular endothelial growth factor (VEGF) gene. In one or more embodiments, the suppression of the expression of the VEGF gene includes the inhibition of normal splicing.

In one or more embodiments, the active ingredient of the pharmaceutical composition of the present disclosure is a low molecular weight compound that is able to reduce the production of VEGF protein from cells. In one or more embodiments, the production of the protein from cells means the secretion of the protein from cells.

The specific examples of the "low molecular weight compound that is able to suppress the expression of the VEGF gene in cells or to reduce the production of VEGF protein from cells" will be described later.

In the present disclosure, the suppression of the expression of the gene and the reduction in the production of the protein are defined as follows. In one or more embodiments, when the low molecular weight compound is added to at least one of known in vitro and in vivo assay systems, it is possible to suppress (or reduce) the transcription or translation of the gene or the production of the protein to 90% or less, 80% or less, 70% or less, 60% or less, 50% or less, 40% or less, 30% or less, 20% or less, or 10% or less, compared to the control in which the low molecular weight compound is not added.

In one or more embodiments, the active ingredient of the pharmaceutical composition of the present disclosure is a low molecular weight compound that further has the ability to inhibit the phosphorylation activity of a plurality of kinases. In the present disclosure, the ability to inhibit the kinases is defined as follows. In one or more embodiments, when the low molecular weight compound is added to at least one of known in vitro and in vivo assay systems for studying the inhibition of protein phosphorylation activity, it is possible to inhibit the protein phosphorylation activity to 60% or less, 50% or less, 40% or less, 30% or less, 20% or less, or 10% or less, compared to the control in which the low molecular weight compound is not added. In one or more embodiments, the amount of the compound added to the assay system is 0.01 to 10 µM. In one or more embodiments, the assay of the inhibition of protein phosphorylation activity may be in vitro and/or in vivo assay disclosed in WO 2010/010797.

In another aspect, the present disclosure relates to a method for preventing, improving, inhibiting the development of, and/or treating neovascular diseases. The method includes administering the pharmaceutical composition of the present disclosure to a subject. In yet another aspect, the present disclosure relates to the use of the pharmaceutical composition of the present disclosure in the method for preventing, improving, inhibiting the development of, and/or treating neovascular diseases or disorders of the present disclosure.

Further, in one or more embodiments, the present disclosure relates to the use of a low molecular weight compound in the manufacture of the pharmaceutical composition for neovascular diseases of the present disclosure. The low molecular weight compound is able to suppress the expression of the VEGF gene in cells or to reduce the production of VEGF protein from cells.

In one or more embodiments, the "pharmaceutical composition" of the present disclosure may have a dosage form suitable for administration by using the known formulation technology. Specifically, the pharmaceutical composition can be administered orally in dosage forms (but not limited to) such as tablets, capsules, granules, powder, pills, troche, syrups, and liquid formulations. Alternatively, the pharmaceutical composition can be administered parenterally in dosage forms (but not limited to) such as injection, liquid formulations, aerosol, suppositories, patches, cataplasm, lotions, liniments, ointments, and eye drops. These formulations can be produced by a known method using additives (but not limited to) such as excipients, lubricants, binders, disintegrators, stabilizers, corrigents, and diluents.

Examples of the excipient include (but are not limited to) the following: starches such as starch, potato starch, and corn starch; lactose; crystalline cellulose; and calcium hydrogen phosphate. Examples of the lubricant include (but are not limited to) the following: ethyl cellulose; hydroxypropyl cellulose; hydroxypropyl methylcellulose; shellac; talc; carnauba wax; and paraffin. Examples of the binder include (but are not limited to) the following: polyvinyl pyrrolidone; macrogol; and the compounds similar to those given as examples of the excipient. Examples of the disintegrator include (but are not limited to) the following: the compounds similar to those given as examples of the excipient; and chemically modified starches and celluloses such as croscarmellose sodium, sodium carboxymethyl starch, and cross-linked polyvinylpyrrolidone. Examples of the stabilizer include (but are not limited to) the following: parahydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. Examples of the corrigent include (but are not limited to) commonly used sweeteners, acidulants, and flavors.

The preparation of a liquid formulation may use (but are not limited to) ethanol, phenol, chlorocresol, purified water, or distilled water as a solvent, and may also use a surface-active agent or an emulsifying agent as needed. Examples of the surface-active agent or the emulsifying agent include (but are not limited to) polysorbate 80, polyoxyl 40 stearate, and lauromacrogol.

The method for using the pharmaceutical composition of the present disclosure may differ depending on symptoms, ages, administration methods, etc. The method allows the pharmaceutical composition to be intermittently or continuously administered (but not limited to) orally, endermically, submucosally, subcutaneously, intramuscularly, intravascularly, intracerebrally, or intraperitoneally so that the concentration of the active ingredient, i.e., the "low molecular weight compound that is able to suppress the expression of the VEGF gene in cells or to reduce the production of VEGF protein from cells" in the body is in the range of 100 nM to 1 mM. In a non-limiting embodiment, for oral administration, the pharmaceutical composition may be administered to a subject (e.g., an adult human) in a dosage of 0.01 mg (preferably 0.1 mg) to 2000 mg (preferably 500 mg and more preferably 100 mg), which is expressed in terms of the "low molecular weight compound that is able to suppress the expression of the VEGF gene in cells or to reduce the production of VEGF protein from cells", once or several times a day based on the symptom. In a non-limiting embodiment, for intravenous administration, the pharmaceutical composition may be administered to a subject (e.g., an adult human) in a dosage of 0.001 mg (preferably 0.01 mg) to 500 mg (preferably 50 mg) once or several times a day based on the symptom.

The present disclosure may relate to one or more embodiments below.
[a1] A pharmaceutical composition for neovascular diseases, containing as an active ingredient a low molecular weight compound that is able to suppress the expression of the VEGF gene in cells or to reduce the production of VEGF protein from cells.
[a2] A pharmaceutical composition for preventing, improving, inhibiting the development of, and/or treating neovascular diseases, containing as an active ingredient a low molecular weight compound that is able to suppress the expression of the VEGF gene in cells or to reduce the production of VEGF protein from cells.
[a3] The pharmaceutical composition according to [a1] or [a2], wherein the neovascular diseases include retinal and choroidal neovascular diseases and cancer.
[a4] The pharmaceutical composition according to any one of [a1] to [a3], wherein the retinal and choroidal neovascular diseases are selected from the group consisting of age-related macular degeneration, glaucoma, occlusion of the retinal vein, and diabetic retinopathy.
[a5] The pharmaceutical composition according to any one of [a1] to [a4], wherein the low molecular weight compound has the ability to inhibit the phosphorylation activity of a plurality of kinases.
[a6] A method for preventing, improving, inhibiting the development of, and/or treating neovascular diseases, including:
   administering the pharmaceutical composition according to any one of [a1] to [a5] to a subject.
[a7] Use of the pharmaceutical composition according to any one of [a1] to [a5] in a method for preventing, improving, inhibiting the development of, and/or treating neovascular diseases.
[a8] Use of a low molecular weight compound in treatment of neovascular diseases, the low molecular weight compound being able to suppress the expression of the VEGF gene in cells or to reduce the production of VEGF protein from cells.
[a9] Use of a low molecular weight compound in the manufacture of a pharmaceutical composition for neovascular diseases, the low molecular weight compound being able to suppress the expression of the VEGF gene in cells or to reduce the production of VEGF protein from cells.
[a10] A method for improving, inhibiting the development of, and/or treating neovascular diseases, including:
   administering a pharmaceutical composition containing a low molecular weight compound to a subject, the low molecular weight compound being able to suppress the expression of the VEGF gene in cells or to reduce the production of VEGF protein from cells.

[Low molecular weight compound that is able to suppress expression of the VEGF gene in cells or to reduce production of VEGF protein from cells]

In one or more embodiments, the "low molecular weight compound that is able to suppress the expression of the VEGF gene in cells or to reduce the production of VEGF protein from cells" in the present disclosure is a compound expressed by the following formula (I) or a prodrug thereof or pharmaceutically acceptable salts thereof 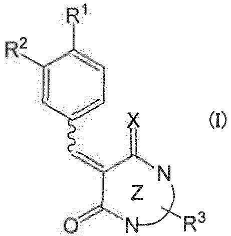
where, in the formula (I), R¹ represents a hydrogen atom, a halogen atom, a hydroxyl group, or a substituted or unsubstituted C₁₋₆ alkyl group,
R² represents a hydrogen atom, a halogen atom, a hydroxyl group, a nitro group (-NO₂), a substituted or unsubstituted C₁₋₆ alkyloxy group, or a substituted or unsubstituted C₁₋₆ alkyl group,
X represents NH or O,
Z represents a 6- or 5-membered monocyclic nitrogen-containing heterocyclic ring, or a bicyclic nitrogen-containing heterocyclic ring formed by the condensation of a 6- or 5-membered nitrogen-containing heterocyclic ring and a 6- or 5-membered aliphatic ring, aromatic ring, or heterocyclic ring,
R³ represents a hydrogen atom, a halogen atom, a hydroxyl group, a substituted or unsubstituted C₁₋₄ alkyl group, a substituted or unsubstituted benzyl or heteroarylmethyl group, a substituted or unsubstituted aryl group, or a substituted or unsubstituted heteroaryl group, and
a bond represented by a wavy line indicates a cis form, a trans form, or a mixture of a cis form and a trans form.

In one or more embodiments, the compound expressed by the formula (I) is a mixture of isomers or an isolated compound when an asymmetric carbon atom and/or a stereoisomer is present.

In one or more embodiments, the "prodrug" in the present disclosure may be a compound that is easily hydrolyzed in a living organism to regenerate the compound expressed by the formula (I). If a compound has, e.g., a carboxyl group, the prodrug of the compound may be a compound in which the carboxyl group is converted to an alkoxycarbonyl group, a compound in which the carboxyl group is converted to an alkylthiocarbonyl group, or a compound in which the carboxyl group is converted to an alkylaminocarbonyl group. Moreover, if a compound has, e.g., an amino group, the prodrug of the compound may be a compound in which the amino group is substituted with an alkanoyl group to form an alkanoylamino group, a compound in which the amino group is substituted with an alkoxycarbonyl group to form an alkoxycarbonylamino group, a compound in which the amino group is converted to an acyloxymethylamino group, or a compound in which the amino group is converted to hydroxylamine. Further, if a compound has, e.g., a hydroxyl group, the prodrug of the compound may be a compound in which the hydroxyl group is substituted with the acyl group to form an acyloxy group, a compound in which the hydroxyl group is converted to a phosphoric ester, or a compound in which the hydroxyl group is converted to an acyloxymethyloxy group. The alkyl portion of the group used for the conversion to the prodrug may be an alkyl group, as will be described later. The alkyl group may be substituted (e.g., with an alkoxy group having 1 to 6 carbon atoms). In one or more embodiments, e.g., when the prodrug is a compound obtained by converting the carboxyl group to an alkoxycarbonyl group, the compound may include lower alkoxycarbonyl (e.g., having 1 to 6 carbon atoms) such as methoxycarbonyl and ethoxycarbonyl, or lower alkoxycarbonyl (e.g., having 1 to 6 carbon atoms) that is substituted with an alkoxy group such as methoxymethoxycarbonyl, ethoxymethoxycarbonyl, 2-methoxyethoxycarbonyl, 2-methoxyethoxymethoxycarbonyl, and pivaloyloxymethoxycarbonyl.

In one or more embodiments, examples of the "C₁₋₆ alkyl group" in the present disclosure include the following: a methyl group; an ethyl group; a 1-propyl group; a 2-propyl group; a 2-methyl-1-propyl group; a 2-methyl-2-propyl group; a 1-butyl group; a 2-butyl group; a 1-pentyl group; a 2-pentyl group; a 3-pentyl group; a 2-methyl-1-butyl group; a 3-methyl-1-butyl group; a 2-methyl-2-butyl group; a 3-methyl-2-butyl group; a 2,2-dimethyl-1-propyl group; a 1-hexyl group; a 2-hexyl group; a 3-hexyl group; a 2-methyl-1-pentyl group; a 3-methyl-1-pentyl group; a 4-methyl-1-pentyl group; a 2-methyl-2-pentyl group; a 3-methyl-2-pentyl group; a 4-methyl-2-pentyl group; a 2-methyl-3-pentyl group; a 3-methyl-3-pentyl group; a 2,3-dimethyl-1-butyl group; a 3,3-dimethyl-1-butyl group; a 2,2-dimethyl-1-butyl group; a 2-ethyl-1-butyl group; a 3,3-dimethyl-2-butyl group; and a 2,3-dimethyl-2-butyl group.

In one or more embodiments, the "C₁₋₆ alkyl group" of the "C₁₋₆ alkyloxy group" in the present disclosure may be any of the examples of the "C₁₋₆ alkyl group" as defined above.

Unless otherwise noted, the number of substituents concerning "substituted or unsubstituted" in the present disclosure may be one or more than one, and the substituents may be either the same or different. In one or more embodiments, examples of the substituent include the following: a halogen atom; a cyano group; a trifluoromethyl group; a nitro group; a hydroxyl group; a methylenedioxy group; a lower alkyl group; a lower alkoxy group; a benzyloxy group; a lower alkanoyloxy group; an amino group; a mono-lower alkylamino group; a di-lower alkylamino group; a carbamoyl group; a lower alkylaminocarbonyl group; di-lower alkylaminocarbonyl group; a carboxyl group; a lower alkoxycarbonyl group; a lower alkylthio group; a lower alkylsulfinyl group; a lower alkylsulfonyl group; a lower alkanoylamino group; and a lower alkylsulfonamide group. In one or more embodiments, the halogen atom may be, e.g., a fluorine atom, a chlorine atom, a bromine atom, or an iodine atom. In one or more embodiments, the lower alkyl may be any of the examples of the "C₁₋₆ alkyl group" as defined above.

In one or more embodiments, the "heterocyclic ring" in the present disclosure may be a non-aromatic ring or an aromatic ring that contains 1 to 3 hetero atoms per ring as ring member atoms. The heterocyclic ring in the present disclosure may have a double bond in the ring. The heterocyclic ring in the present disclosure may have either one ring or two or more rings that are condensed together. In one or more embodiments, the "hetero atom" in the present disclosure may be a sulfur atom, an oxygen atom, or a nitrogen atom. When the heterocyclic ring contains a plurality of hetero atoms, they are the same or different.

In one or more embodiments, the aryl group and the "aryl group" of the heteroarylmethyl group and the heteroaryl group in the present disclosure may be an aryl group having 10 or less carbon atoms such as a phenyl group or a naphthyl group.

The "pharmaceutically acceptable salt" in the present disclosure includes a pharmacologically and/or medically acceptable salt, and may be, e.g., an inorganic acid salt, an organic acid salt, an inorganic base salt, an organic base salt, or an acidic or basic amino acid salt.

Preferred examples of the inorganic acid salt include the following: hydrochloride; hydrobromate; sulfate; nitrate; and phosphate. Preferred examples of the organic acid salt include the following: acetate; succinate; fumarate; maleate; tartrate; citrate; lactate; stearate; benzoate; methanesulfonate; and p-toluenesulfonate.

Preferred examples of the inorganic base salt include the following: alkali metal salts such as sodium salt and potassium salt; alkaline-earth metal salts such as calcium salt and magnesium salt; aluminum salts; and ammonium salts. Preferred examples of the organic base salt include the following: diethylamine salt; diethanolamine salt; meglumine salt; and N,N'-dibenzylethylenediamine salt.

Preferred examples of the acidic amino acid salt include aspartate and glutamate. Preferred examples of the basic amino acid salt include arginine salt, lysine salt, and ornithine salt.

The "salt of the compound" in the present disclosure may include a hydrate that can be formed by allowing the compound to stand in the air so that it absorbs water. Moreover, the "salt of the compound" in the present disclosure may also include a solvate that can be formed by letting the compound absorb some type of solvent.

In one or more embodiments, R¹ of the formula (I) represents a hydrogen atom, a halogen atom, or a hydroxyl group, or represents a hydrogen atom or a hydroxyl group, or represents a hydroxyl group.

In one or more embodiments, R² of the formula (I) represents a hydrogen atom, a halogen atom, a nitro group, or a substituted or unsubstituted C₁₋₆ alkyloxy group, or represents a hydrogen atom, a nitro group, or a substituted or unsubstituted C₁₋₃ alkyloxy group, or represents a nitro group or a methoxy group.

In one or more embodiments, Z of the formula (I) represents, together with three carbon atoms and two nitrogen atoms of the formula (I), the formation of a 6- or 5-membered monocyclic nitrogen-containing heterocyclic ring or the formation of a bicyclic nitrogen-containing heterocyclic ring by the condensation of a 6- or 5-membered nitrogen-containing heterocyclic ring and a 6- or 5-membered aliphatic ring, aromatic ring, or heterocyclic ring.

In one or more embodiments, R³ of the formula (I) represents one to four substituents or one substituent of the ring Z. If the formula (I) has more than one R³, they may independently the same or different.

In one or more embodiments, R³ of the formula (I) represents a hydrogen atom, a halogen atom, a hydroxyl group, a substituted or unsubstituted C₁₋₄alkyl group, a substituted or unsubstituted benzyl group, a substituted or unsubstituted aryl group, or a phenyl group or a trifluoromethyl group that is substituted with a halogen.

In one or more embodiments, the compound of the formula (I) is a compound expressed by the following formula (II) or (III): 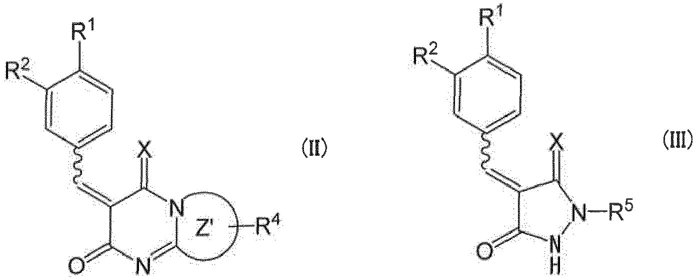
where, in the formulas (II) and (III), R¹, R², and X are the same as those of the formula (I),
in the formula (II), R⁴ represents a hydrogen atom, a halogen atom, a hydroxyl group, a substituted or unsubstituted C₁₋₄ alkyl group, a substituted or unsubstituted benzyl or heteroarylmethyl group, a substituted or unsubstituted aryl group, or a substituted or unsubstituted heteroaryl group,
in the formula (III), Z' represents a 6- or 5-membered monocyclic nitrogen-containing heterocyclic ring, and R⁵ represents a hydrogen atom, a halogen atom, a hydroxyl group, or a substituted or unsubstituted C₁₋₄alkyl group, and
in the formulas (II) and (III), a bond represented by a wavy line indicates a cis form, a trans form, or a mixture of a cis form and a trans form at a desired ratio.

R¹, R², and X of the formula (II) are the same as those of the formula (I), and in one or more embodiments, X represents an oxygen atom.

In one or more embodiments, R⁴ of the formula (II) represents a substituted or unsubstituted C₁₋₄alkyl group, a substituted or unsubstituted benzyl group, a substituted or unsubstituted aryl group, a substituted or unsubstituted aryl group, or a phenyl group that is substituted with one or more halogens.

R¹, R², and X of the formula (III) are the same as those of the formula (I), and in one or more embodiments, X represents NH.

In one or more embodiments, Z' of the formula (III) represents, together with one N atom and one C atom of the formula (III), the formation of a 6- or 5-membered monocyclic nitrogen-containing heterocyclic ring. In one or more embodiments, Z' of the formula (III) represents a 5-membered nitrogen-containing heterocyclic ring, a 5-membered nitrogen-containing heteroaromatic ring, or a 5-membered heteroaromatic ring containing two N atoms and one S atom.

In one or more embodiments, R⁵ represents a hydrogen atom, a halogen atom, a hydroxyl group, or a substituted or unsubstituted C₁₋₄alkyl group, or represents a substituted or unsubstituted C₁₋₄alkyl group, or represents a trifluoromethyl group.

In one or more embodiments, the compound of the formula (I) is expressed by

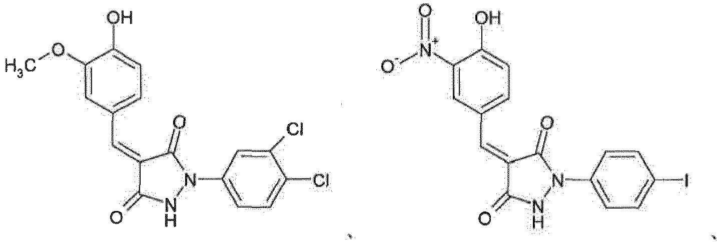

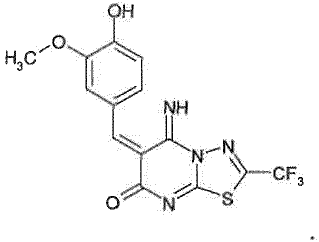

The present disclosure may relate to one or more embodiments below.
[b1] A compound expressed by the following formula (I) or a prodrug thereof or pharmaceutically acceptable salts thereof 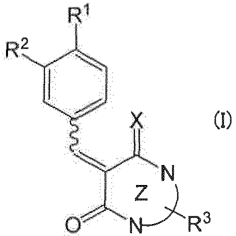 where, in the formula (I), R¹ represents a hydrogen atom, a halogen atom, a hydroxyl group, or a substituted or unsubstituted C₁₋₆alkyl group, R² represents a hydrogen atom, a halogen atom, a hydroxyl group, a nitro group (-NO₂), a substituted or unsubstituted C₁₋₆alkyloxy group, or a substituted or unsubstituted C₁₋₆alkyl group, X represents NH or O, Z represents a 6- or 5-membered monocyclic nitrogen-containing heterocyclic ring, or a bicyclic nitrogen-containing heterocyclic ring formed by the condensation of a 6- or 5-membered nitrogen-containing heterocyclic ring and a 6- or 5-membered aliphatic ring, aromatic ring, or heterocyclic ring, R³ represents a hydrogen atom, a halogen atom, a hydroxyl group, a substituted or unsubstituted C₁₋₄alkyl group, a substituted or unsubstituted benzyl or heteroarylmethyl group, a substituted or unsubstituted aryl group, or a substituted or unsubstituted heteroaryl group, and a bond represented by a wavy line indicates a cis form, a trans form, or a mixture of a cis form and a trans form at a desired ratio.
[b2] A compound expressed by the following formula (II) or (III) or a prodrug thereof or pharmaceutically acceptable salts thereof 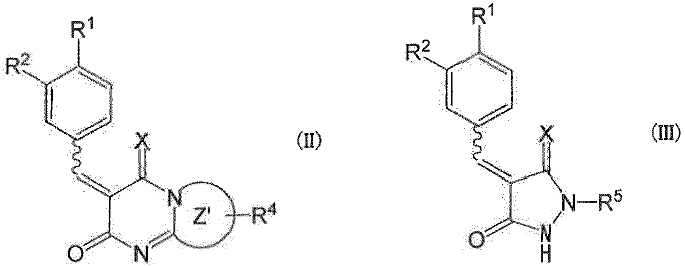 where, in the formulas (II) and (III), R¹, R², and X are the same as those of the formula (I), in the formula (II), R⁴ represents a hydrogen atom, a halogen atom, a hydroxyl group, a substituted or unsubstituted C₁₋₄alkyl group, a substituted or unsubstituted benzyl or heteroarylmethyl group, a substituted or unsubstituted aryl group, or a substituted or unsubstituted heteroaryl group, in the formula (III), Z' represents a 6- or 5-membered monocyclic nitrogen-containing heterocyclic ring, and R⁵ represents a hydrogen atom, a halogen atom, a hydroxyl group, or a substituted or unsubstituted C₁₋₄alkyl group, and a bond represented by a wavy line indicates a cis form, a trans form, or a mixture of a cis form and a trans form at a desired ratio.
[b3] A compound expressed by 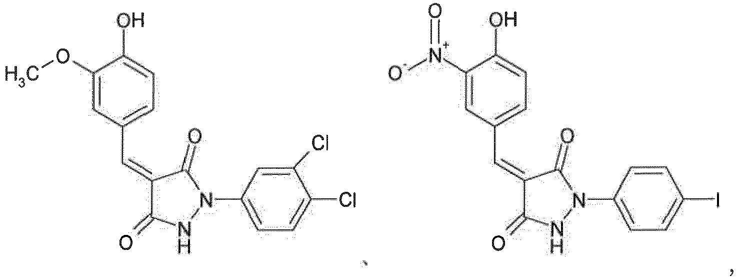 or 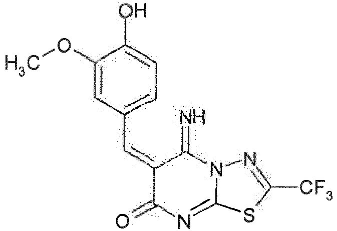
or a prodrug thereof or pharmaceutically acceptable salts thereof.

In one or more embodiments, the compound expressed by the formula (I) or the prodrug thereof or the pharmaceutically acceptable salts thereof may be a low molecular weight compound that is able to suppress the expression of the VEGF gene in cells or to reduce the production of VEGF protein from cells.

In one or more embodiments, the compound expressed by the formula (I) or the prodrug thereof or the pharmaceutically acceptable salts thereof may be an active ingredient of a pharmaceutical composition for neovascular diseases. Therefore, in one aspect, the present disclosure relates to a pharmaceutical composition for neovascular diseases, containing as an active ingredient the compound expressed by the formula (I) or the prodrug thereof or the pharmaceutically acceptable salts thereof. In one or more embodiments, the pharmaceutical composition of this aspect is a pharmaceutical composition for preventing, improving, inhibiting the development of, and/or treating neovascular diseases. A method for using the pharmaceutical composition of this aspect and a method for preventing, improving, inhibiting the development of, and/or treating neovascular diseases with the use of the pharmaceutical composition of this aspect can be the same as those described above.

In one or more embodiments, the compound expressed by the formula (I) or the prodrug thereof or the pharmaceutically acceptable salts thereof further may have the ability to inhibit the phosphorylation activity of a plurality of kinases.

The present disclosure may relate to one or more embodiments below.
[c1] A pharmaceutical composition for neovascular diseases, containing as an active ingredient the compound or the prodrug thereof or the pharmaceutically acceptable salts thereof according to any one of [b1] to [b3].
[c2] A pharmaceutical composition for preventing, improving, inhibiting the development of, and/or treating neovascular diseases, containing as an active ingredient the compound or the prodrug thereof or the pharmaceutically acceptable salts thereof according to any one of [b1] to [b3].
[c3] The pharmaceutical composition according to [c1] or [c2], wherein the neovascular diseases include retinal and choroidal neovascular diseases and cancer.
[c4] The pharmaceutical composition according to any one of [c1] to [c3], wherein the retinal and choroidal neovascular diseases are selected from the group consisting of age-related macular degeneration, glaucoma, occlusion of the retinal vein, and diabetic retinopathy.
[c5] The pharmaceutical composition according to any one of [c1] to [c4],
   wherein the compound or the prodrug thereof or the pharmaceutically acceptable salts thereof according to any one of [b1] to [b3] is a low molecular weight compound that has the ability to inhibit the phosphorylation activity of a plurality of kinases.
[c6] A method for preventing, improving, inhibiting the development of, and/or treating neovascular diseases, including:
   administering the pharmaceutical composition according to any one of [c1] to [c5] or the compound or the prodrug thereof or the pharmaceutically acceptable salts thereof according to any one of [b1] to [b3] to a subject.
[c7] Use of the pharmaceutical composition according to any one of [c1] to [c5] or the compound or the prodrug thereof or the pharmaceutically acceptable salts thereof according to any one of [b1] to [b3] in a method for preventing, improving, inhibiting the development of, and/or treating neovascular diseases.
[c8] Use of the compound or the prodrug thereof or the pharmaceutically acceptable salts thereof according to any one of [b1] to [b3] in the manufacture of a pharmaceutical composition for neovascular diseases.

### Examples

Hereinafter, the present disclosure will be described in more detail by way of examples. These examples are for illustrative purposes only, and the present disclosure is not limited to the examples. All the documents cited in the present disclosure are incorporated herein by reference.

### Production example 1: production of compound 1

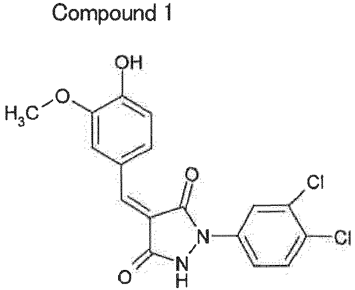

A compound 1 was produced in the following manner.

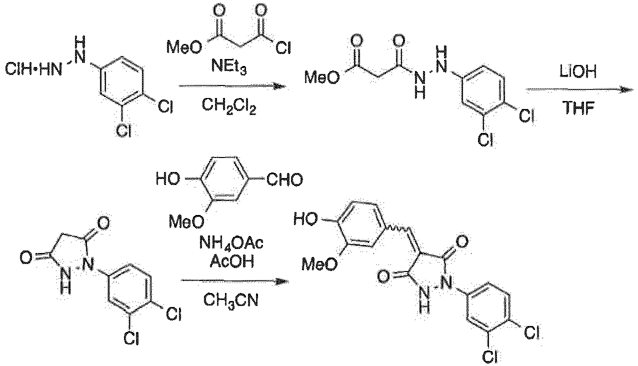

Under an argon atmosphere, triethylamine (1.66 mL, 12.0 mmol, commercial product) and methyl 3-chloro-3-oxopropionate (0.60 mL, 5.56 mmol, commercial product) were successively added at 0°C to a methylene chloride (20 mL) solution of 3,4-dichlorophenylhydrazine hydrochloride (1.06 g, 5.00 mmol, commercial product), and then the solution was stirred for 1 hour. The reaction was stopped by adding 20 mL of water to the reaction mixture, and an extraction was performed using methylene chloride (20 mL × 2). Subsequently, the organic layers were combined, washed with saturated saline, dried over anhydrous sodium sulfate, and filtered. The filtrate was concentrated under reduced pressure. The reaction crude product was purified by silica gel column chromatography (Presep (registered trademark) Silica Gel (HC-N) Type L, manufactured by Wako Pure Chemical Industries, Ltd., hexane/ethyl acetate =1/1). Thus, methyl 3-[2-(3,4-dichlorophenyl)hydrazino]-3-oxopropionate (1.10 g, 3.97 mmol, 79.4%) was obtained as a light yellow solid.

Then, water (0.5 mL) and lithium hydroxide monohydrate (20 mg, 0.48 mmol, commercial product) were added at room temperature to a tetrahydrofuran (2.0 mL) solution of the methyl 3-[2-(3,4-dichlorophenyl)hydrazino]-3-oxopropionate (111 mg, 0.397 mmol) thus obtained. This reaction mixture was stirred at room temperature for 1 hour, and the solvent was distilled under reduced pressure. The resulting solid was mixed with acetonitrile (4.0 mL), vanillin (60.2 mg, 0.396 mmol, commercial product), ammonium acetate (32.0 mg, 0.415 mmol, commercial product), and acetic acid (20 mL, 0.35 mmol, commercial product). After the mixture was stirred by heating at 90°C for 48 hours, the generated orange precipitate was filtered off, washed successively with diethyl ether (3 mL), ethyl acetate (3 mL), water (3 mL), ethyl acetate (3 mL), and diethyl ether (3 mL), and dried under reduced pressure. Consequently, 1-(3,4-dichlorophenyl)-4-(4-hydroxy-3-methoxybenzylidene)pyrazolidine-3,5-dione (91.3 mg, 0.241 mmol, 60.8%) was obtained as an orange solid. Melting point: 260°C (decomp.); ¹H NMR (DMSO- d₆, 400 MHz) δ 3.82-3.87 (br, 3H), 6.82-6.90 (br, 1H), 7.59-7.81 (m, 3H), 7.88-7.99 (br, 1H), 8.00-8.09 (br, 1H), 8.58-8.71 (br, 1H), 10.40-11.40 (br, 1H); HRMS (ESI-) m/z 369.0111 ([M-H]-, C₁₇H₁₁Cl₂N₂O₄-requires 377.0101)

### Production example 2: production of compound 2

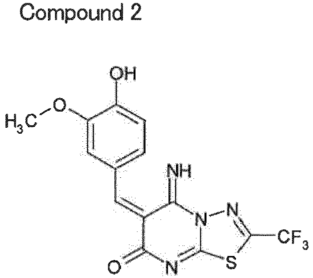

A compound 2 was produced in the following manner.

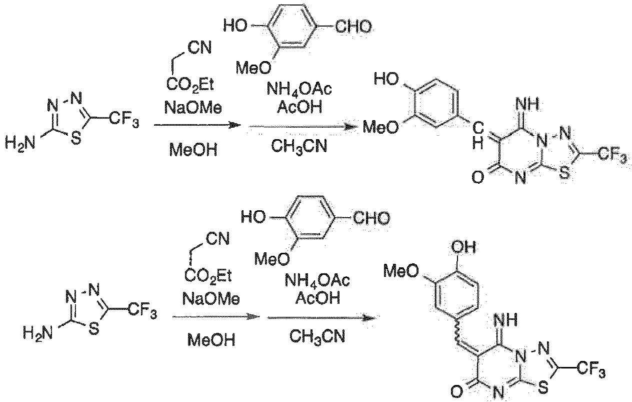

Under an argon atmosphere, ethyl cyanoacetate (10.6 mL, 99.4 mmol, commercial product) and sodium methoxide (10.8 g, 200 mmol, commercial product) were successively added to a methanol (300 mL) solution of 2-amino-5-trifluoromethyl-1,3,4-thiadiazole (16.9 g, 100 mmol, commercial product), and then the solution was stirred at 60°C for 20 hours. The solvent was distilled from the reaction mixture under reduced pressure, so that a white solid (39.2 g) was obtained.

Next, a part (36.0 g) of the white solid was mixed with acetonitrile (800 mL), vanillin (21.3 g, 140 mmol, commercial product), ammonium acetate (10.5 g, 136 mmol, commercial product), and acetic acid (6.3 mL, 110 mmol, commercial product), and the mixture was heated to 90°C. After this reaction mixture was stirred for 1 hour, the generated yellow precipitate was filtered off. The resulting solid was dissolved in water (500 mL) and ethyl acetate (1 L), and then the organic layers were separated and concentrated. Subsequently, the crude product was divided into ten parts, and each of them was purified by silica gel column chromatography (Biotage (trade name) ZIP sphere cartridge [silica] 120 g, methylene chloride/methanol = 97/3). Thus, 6-(4-hydroxy-3-methoxybenzylidene)-5-imino-2-(trifluoromethyl)-5,6-dihydro-7H-[1,3,4 ]thiadiazolo[3,2-a]pyrimidin-7-one (12.0 g, 32.4 mmol, 35.3%) was obtained as a yellow solid.
Melting point: 224-225°C; ¹H NMR (DMSO- d₆, 500 MHz) δ 3.84 (s, 3H), 7.01 (d, J = 8.5 Hz, 1H), 7.56 (dd, J = 8.5, 2.5 Hz, 1H), 7.75 (d, J = 2.5 Hz, 1H), 8.47 (s, 1H); ¹⁹F NMR (DMSO- d₆, 376 MHz) δ -58.8- -58.5 (br); HRMS (ESI-) m/z 369.0274 ([M-H]-, C₁₄H₈F₃N₄O₃S- requires 369.0275)

### Compound 3

A compound 3 expressed by the following formula was a product (manufactured by InterBioScreen Ltd.) on the market.

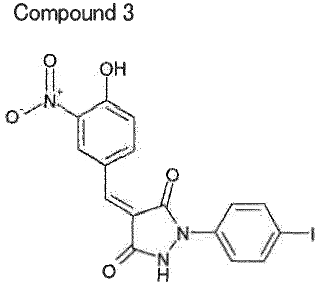

### [Experiment 1: Evaluation of effect of inhibiting VEGF expression by compounds 1 to 3]

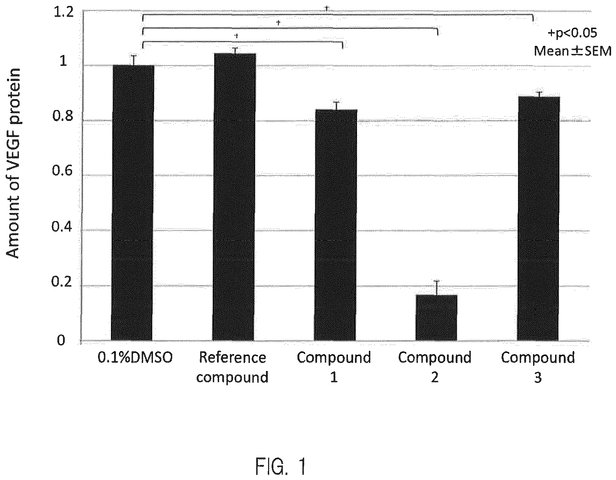

The synthesized compounds 1 to 3 and the following reference compound were administered to human retinal pigment epithelial cells (ARPE-19 cell line). After 3 days of cultivation, each culture medium was subjected to ELISA to determine a vascular endothelial growth factor (VEGF). The details of the experiment are as follows. FIG. 1 illustrates the results.

### [Reference compound]

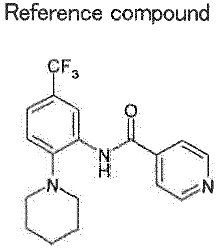

The reference compound inhibits SRPK (kinase) and has antiviral activity (see WO 2005/063293). The reference compound has been reported to suppress retinal neovascularization (Nowak DG, Bates DO et al., J. Bio. Chem. 2010).

### [Experimental conditions]

A retinal pigment epithelial cell line, i.e., ARPE-19 was used. The ARPE-19 was cultured in Ham's F-12/DMEM medium containing 10% fetal bovine serum and 1% penicillin-streptomycin solution. The cells were seeded on a 24 well plate at 1.0 × 10⁴ cell/well. On the next day, the culture medium was replaced with 1% fetal bovine serum. Further, the culture medium was removed 24 hours later and replaced with a culture medium (containing 1% fetal bovine serum) to which the compound (10 µM) and TGF-β (1 nM) were added. The culture medium was replaced every 24 hours in the same manner. Then, the culture medium was collected after 72 hours had passed from the administration of the compound. An ELISA kit (manufactured by R&D Systems, Inc.) was used to measure the amount of VEGF protein in the culture medium. The absorbance was measured by an ARVO X5 multiplate reader.

As can be seen from FIG. 1, the results confirmed that the amount of VEGF protein was reduced in the samples to which compounds 1 to 3 were added. Such a reduction in the amount of VEGF protein was particularly significant when compound 2 was administered.

### [Experiment 2: Evaluation of effect of inhibiting CNV formation by compounds 1 to 3]

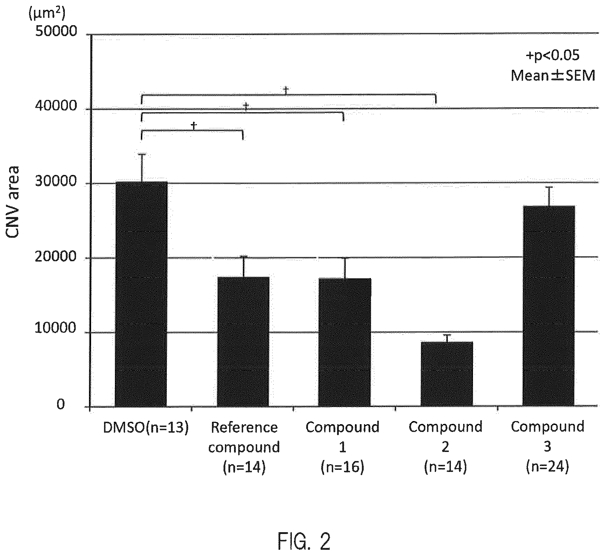

The synthesized compounds 1 to 3 and the above reference compound were administered to model mice with choroidal neovascularization (CNV) by intravitreal injection, and CNV inhibition was evaluated. The details of the experiment are as follows. FIG. 2 illustrates the results.

### [CNV model mice]

100 mg/kg of ketamine and 10 mg/kg of xylazine were intraperitoneally injected into 6- to 8-week-old C57B/6J mice (Japan SLC, Inc.), and the mice were placed under anesthesia. Then, eyedrops (SANDOL P) containing tropicamide (5 mg/ml) and phenylephrine (5 mg/ml) were used to dilate the pupil. Any four points of the fundus were irradiated with a 532 nm argon laser (100 mW, 0.1 s, 75 µm) to form new blood vessels. The compound (20 pmol) was intraocularly injected immediately after the laser irradiation, and an ofloxacin (Tarivid) ophthalmic ointment was applied to the corneal surface to prevent infection and drying.

### [Experimental conditions]

100 mg/kg of ketamine and 10 mg/kg of xylazine were intraperitoneally injected into the mice after 7 days had passed from the laser irradiation, and the mice were placed under anesthesia. Then, the mice underwent thoracotomy and were perfused with FITC-dextran (50 mg/ml; Life Technologies Japan Ltd.) through the left ventricle. After the perfusion, the eyes were enucleated and fixed in 4% paraformaldehyde. After the fixation, the corneas, crystalline lenses, and retinas were removed, and the flat mount of the choroid was obtained. CNV was imaged with a fluorescence microscope (BZ-9000; Keyence Corporation) so that the area of CNV was measured.

As can be seen from FIG. 2, the results confirmed that CNV formation was reduced in the samples to which compounds 1 to 3 were added. Such a reduction in CNV formation was significant when compounds 1 and 2 were administered, and the effect of compound 2 was more prominent.

### [Experiment 3: Confirmation of concentration dependence of effect of inhibiting CNV formation]

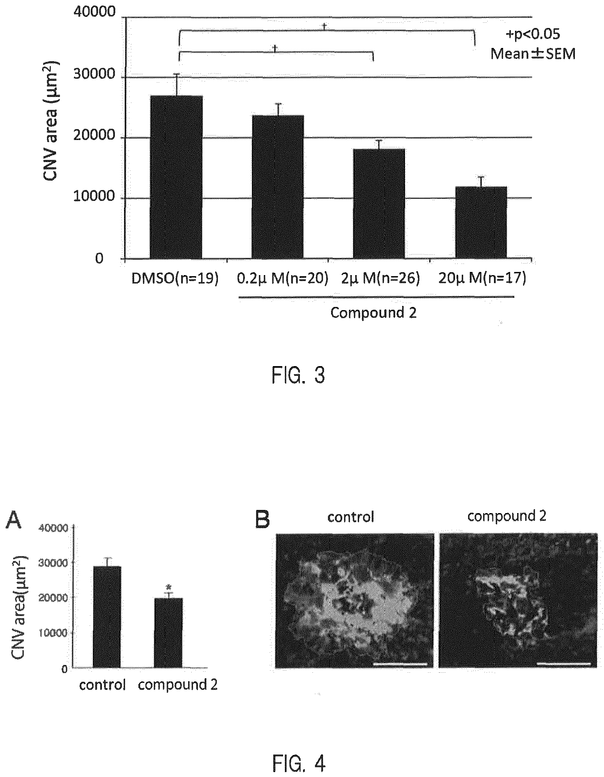

An experiment was performed in the same manner as Experiment 2 by administering the compound 2 at different concentrations (0.2 µM, 2 µM, 20 µM), and CNV inhibition was evaluated. FIG. 3 illustrates the results.

As can be seen from FIG. 3, the results confirmed that compound 2 reduced CNV formation in a concentration-dependent manner.

As described above, compounds 1 to 3 can reduce the amount of VEGF protein to be produced and inhibit CNV formation. Therefore, compounds 1 to 3 are useful in the field of medication, treatment, etc. of neovascular diseases.

[Experiment 4: Preparation of ophthalmic ointment and evaluation of effect of inhibiting CNV formation by using the ophthalmic ointment]

### [Preparation of ophthalmic ointment]

An ophthalmic ointment with the following composition was prepared. Composition (per 100 g):
10 g of compound 2;
60 g of liquid paraffin; and
30 g of white petrolatum.

### [Experimental conditions]

An experiment was performed in the same manner as Experiment 2 except that the ophthalmic ointment was applied in a dose of 7 µL three times a day, instead of the intravitreal injection, and CNV inhibition was evaluated. Moreover, an ophthalmic ointment containing only a base material was used as a control, and the same experiment was performed. FIG. 4 illustrates the results. A of FIG. 4 is a graph that indicates the area of CNV. B of FIG. 4 shows an example of fluorescence microscopy images of the choroid. As can be seen from A and B of FIG. 4, the results confirmed that compound 2 reduced CNV formation.

## Claims

1. A pharmaceutical composition for neovascular diseases, containing as an active ingredient a low molecular weight compound that is able to suppress expression of theVEGF gene in cells or to reduce production of VEGF protein from cells.

2. The pharmaceutical composition according to claim 1, wherein the neovascular diseases include retinal and choroidal neovascular diseases and cancer.

3. The pharmaceutical composition according to claim 2, wherein the retinal and choroidal neovascular diseases include age-related macular degeneration, glaucoma, occlusion of the retinal vein, and diabetic retinopathy.

4. The pharmaceutical composition according to any one of claims 1 to 3, wherein the low molecular weight compound has an ability to inhibit phosphorylation activity of a plurality of kinases.

5. A pharmaceutical composition for neovascular diseases, containing as an active ingredient a compound expressed by the following formula (I) or a prodrug thereof or pharmaceutically acceptable alts thereof 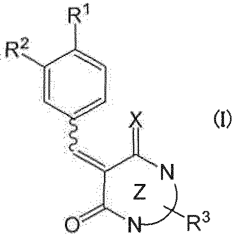
where, in the formula (I), R¹ represents a hydrogen atom, a halogen atom, a hydroxyl group, or a substituted or unsubstituted C₁₋₆alkyl group,
R² represents a hydrogen atom, a halogen atom, a hydroxyl group, a nitro group (-NO₂), a substituted or unsubstituted C₁₋₆alkyloxy group, or a substituted or unsubstituted C₁₋₆alkyl group,
X represents NH or O,
Z represents a 6- or 5-membered monocyclic nitrogen-containing heterocyclic ring, or a bicyclic nitrogen-containing heterocyclic ring formed by condensation of a 6-or 5-membered nitrogen-containing heterocyclic ring and a 6- or 5-membered aliphatic ring, aromatic ring, or heterocyclic ring,
R³ represents a hydrogen atom, a halogen atom, a hydroxyl group, a substituted or unsubstituted C₁₋₄alkyl group, a substituted or unsubstituted benzyl or heteroarylmethyl group, a substituted or unsubstituted aryl group, or a substituted or unsubstituted heteroaryl group, and
a bond represented by a wavy line indicates a cis form, a trans form, or a mixture of a cis form and a trans form at a desired ratio.

6. The pharmaceutical composition according to claim 5, wherein the low molecular weight compound of the formula (I) is a compound expressed by the following formula (II) or (III): 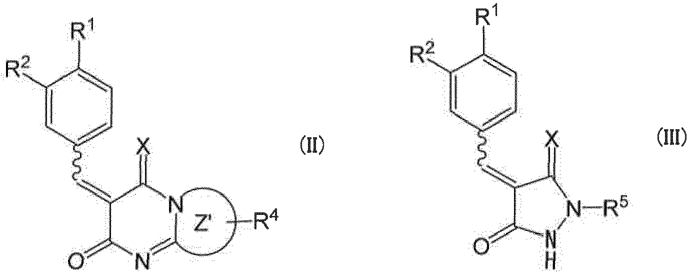
where, in the formulas (II) and (III), R¹, R², and X are the same as those of the formula (I),
in the formula (II), R⁴ represents a hydrogen atom, a halogen atom, a hydroxyl group, a substituted or unsubstituted C₁₋₄alkyl group, a substituted or unsubstituted benzyl or heteroarylmethyl group, a substituted or unsubstituted aryl group, or a substituted or unsubstituted heteroaryl group,
in the formula (III), Z' represents a 6- or 5-membered monocyclic nitrogen-containing heterocyclic ring, and R⁵ represents a hydrogen atom, a halogen atom, a hydroxyl group, or a substituted or unsubstituted C₁₋₄alkyl group, and
in the formulas (II) and (III), a bond represented by a wavy line indicates a cis form, a trans form, or a mixture of a cis form and a trans form at a desired ratio.

7. The pharmaceutical composition according to claim 5, wherein the compound of the formula (I) is selected from the group consisting of 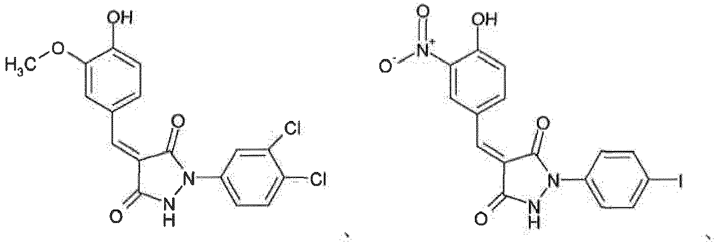 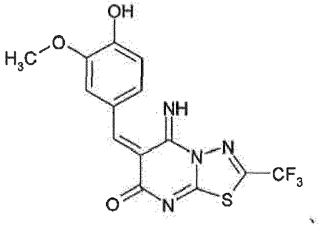 and combinations thereof.

8. Use of a low molecular weight compound in treatment of neovascular diseases, the low molecular weight compound being able to suppress expression of the VEGF gene in cells or to reduce production of VEGF protein from cells.

9. Use of a low molecular weight compound in manufacture of a pharmaceutical composition for neovascular diseases, the low molecular weight compound being able to suppress expression of the VEGF gene in cells or to reduce production of VEGF protein from cells.

10. A method for improving, inhibiting development of, and/or treating neovascular diseases, comprising:
administering a pharmaceutical composition containing a low molecular weight compound as an active ingredient to a subject, the low molecular weight compound being able to suppress expression of the VEGF gene in cells or to reduce production of VEGF protein from cells.
